# EUROPEAN PATENT SPECIFICATION

(11) **EP 3 319 932 B1**
(45) Date of publication and mention of the grant of the patent: **06.05.2020**
(21) Application number: 16736193.0
(22) Date of filing: 08.07.2016
(51) Int. Cl.: C07C 51/43, C07C 59/185

(54) **PROCESS FOR THE ISOLATION OF LEVULINIC ACID**
VERFAHREN ZUR ISOLIERUNG VON LEVULINSÄURE
PROCÉDÉ D'ISOLATION D'ACIDE LÉVULINIQUE

(30) Priority: 10.07.2015 EP 15176322
(43) Date of publication of application: 16.05.2018
(73) Proprietor: GFBiochemicals IP Assets B.V., 6163 JT Geleen (NL)
(72) Inventor: RIJKE, DE, Aris, Valletta VLT 1467 (MT); SANTORO, Donato, Valletta VLT 1467 (MT); PARTON, Rudy Francois Maria Jozef, Valletta VLT 1467 (MT)
(74) Representative: Renkema, Jaap
(86) International application number: PCT/EP2016/066236
(87) International publication number: WO 2017/009211

(56) References cited:
- CN-A- 104 447 285
- US-A- 5 981 810
- US-A1- 2014 316 161

## Description

The present invention is directed to a process for the isolation of levulinic acid.
Levulinic acid, or 4-oxopentanoic acid, is an organic compound with the formula CH₃C(O)CH₂CH₂CO₂H. It can be obtained by degradation of cellulose that is present in lignocellulosic feedstock, for example in agricultural waste products, waste from the paper industry or municipal waste.
Levulinic acid is a precursor to pharmaceuticals, plasticizers, and various other additives. Also biofuels, such as methyltetrahydrofuran, gamma-valerolactone, and ethyl levulinate, can be prepared from levulinic acid.
In the prior art several processes for the production of levulinic acid are described. Levulinic acid can, for example, be produced by acid catalyzed hydrolysis of C6 carbohydrate-containing feedstocks. The production of levulinic acid by acid catalyzed hydrolysis of C6 carbohydrate-containing feedstocks is described e.g. in WO2010/030617, US2010/312006, US2014/316161, US5,608,105, US4,897,497 and US6,054,611. Another process for the production of levulinic acid is a process that produces levulinic acid from furfuryl alcohol or its esters. An example of such a process is described in US 4,236,021. Furfurylalcohol is converted at 125 °C to butyl levulinate in the presence of an alcohol, such as butylalcohol, with HCl as catalyst. Butyl levulinate is isolated via distillation. After hydrolysis in an aqueous environment levulinic acid is obtained.
A third process for the production of levulinic acid is from diethyl acetyl succinate as, for example, described in US5,189,215. Diethyl acetyl succinate can be made from diethyl maleate, which in general is derived from petrochemical based maleic acid anhydride.

During the conversion of C6 carbohydrate-containing feedstocks by acid catalyzed hydrolysis formic acid is produced as a by-product of levulinic acid. Since selectivity is below 100% also many other side products are made. That makes that a process for the production of levulinic acid from C6 carbohydrate-containing feedstocks will generate a complex mixture wherefrom levulinic acid has to be recovered and purified.

During the production of levulinic acid the recovery and purification is, according to the prior art, most preferably performed via fractional distillation; as is for example described in WO2015/007602. Further, crystallization from solution is often used for the crystallization of organic compounds. Crystallization of levulinate from a solution can be seen for instance in CN 104447285. Typical of crystallization from solution is that the solvent is the major compound in the solution that is fed to the crystallization from solution. However, a disadvantage of crystallization from solution is that the product, e.g. levulinic acid crystallizes in a solvent and recovery of the solvent is required.
The further purification of levulinic acid by the use of distillation and/or crystallization is, for example, mentioned in US-2,305,738, US-2,029,412, US-2,684,982 and US-2,349,514. In these documents crystallization is described as a way to further purify a levulinic acid solution with a purity of over 90 wt%. In the mentioned documents it is not specified what type of crystallization can be used.
Another existing crystallization method is melt crystallization. In melt crystallization the crystallizing component is present in a higher amount in the composition that is fed to melt crystallization than all of the other components together. Melt crystallization however is considered very unpredictable by the person skilled in the art. This is because the result of melt crystallization does not only depend on the freezing point of the product, the nature of impurities and whether the impurities form an eutectic mixture with the product, but also on the structure of the crystals formed and therefore on their tendency to occlude impurities. Moreover, the size and productivity of the crystallization depend on the rate of crystal formation without the occlusion of those impurities. In a review of Wynn (Separate organics by melt Crystallization, Chemical Engineering Progress, March 1992, 52-60) was stated "Unfortunately, in melt crystallization, the critical steps are rate dependent. They cannot be predicted accurately from theory. Laboratory or pilot plant data must be generated before even process feasibility can be established".
With the processes according to the prior art it is difficult to obtain levulinic acid with a predetermined purity. A predetermined purity is a purity level of levulinic acid of 85-100 wt%, comprising at most 1 wt% of formic acid, at most 1 wt% of angelica lactone, at most 15 wt% of solvent and at most 5 wt% of impurities, based on the total weight of the levulinic acid. Angelica lactone is the cyclic dehydration product of levulinic acid. It is also called 4-hydroxy-3-pentenoic acid γ-lactone or 5-methyl-2(3H)-furanone.

The inventors now surprisingly discovered that levulinic acid with a predetermined purity can be obtained by melt crystallization.
The process according to the invention comprises the following steps:
a. Performing acid catalyzed hydrolysis of a C6 carbohydrate-containing feedstock to obtain reaction product X,
b. Purifying of reaction product X to provide a composition 1, comprising at least 75 wt% levulinic acid, at most 15 wt% solvent and at most 10 wt% impurities, based on the total weight of composition 1,
c. Feeding composition 1 to a melt crystallization cycle,
d. Repeating the melt crystallization cycle as many times as necessary to obtain as composition 2 levulinic acid with the predetermined purity, and
e. Isolating the levulinic acid with the predetermined purity.

Surprisingly, a levulinic acid with a predetermined purity can be obtained by using melt crystallization for the isolation of levulinic acid from composition 1.
An advantage of the process according to the invention is that also a good separation can be obtained between formic acid and levulinic acid and that a levulinic acid can be obtained which is substantially free of formic acid. Substantially free of formic acid here means that less than 0.5 wt% of formic acid is present in the levulinic acid.

The process according to the invention starts with acid catalyzed hydrolysis of a C6 carbohydrate-containing feedstock to obtain reaction product X.
A C6-carbohydrate is herewith defined as a compound consisting of carbon (C), hydrogen (H) and oxygen (O) atoms. In its monomeric form it comprises 6 carbon atoms and has a hydrogen atom:oxygen atom ratio of 2:1.
A C6-carbohydrate-containing feedstock is for example a feedstock comprising monosaccharides, such as fructose, mannose and glucose; disaccharides such as saccharose and lactose and polysaccharides such as starch and cellulose. A C6-carbohydrate-containing feedstock can also be a lignocellulosic feedstock, which comprises cellulose, hemicellulose and lignin. Examples of lignocellulosic feedstock are wood; wood processing side products such as saw dust, wood chippings and wood shavings; grass; cereals, algae; tree bark; hay; straw; leaves and paper pulp. For the purpose of this application a C6 carbohydrate-containing feedstock comprises at least 20 wt% C6 carbohydrates.

The feedstock is treated by acid catalyzed hydrolysis. Various acid catalysts are suitable for use during acid catalyzed hydrolysis. These include, but are not limited to, inorganic acids such as sulfuric acid, fluorosulfonic acid, hydrochloric acid, nitric acid, phosphoric acid, benzenesulfonic acid, phosphotungstic acid, phosphomolybdic acid; organic acids such as p-toluene sulfonic acid, trifluoromethanesulfonic acid, 1,1,2,2-tetrafluoroethanesulfonic acid, 1,1,2,3,3,3-hexafluoropropanesulfonic acid, and mixtures thereof; acidic-ion exchange resins; Brönsted acid catalysts such as bismuth triflate, yttrium triflate, ytterbium triflate, neodymium triflate, lanthanum triflate, scandium triflate, zirconium triflate, and Zn(BF₄)₂; fluorinated sulfonic acid polymers; metal salts of acid such as metal sulfonates, metal sulfates, metal trifluoroacetates, metal triflates; heteropolyacids; and perfluoroalkylsulfonic acids.

During the acid catalyzed hydrolysis of a C6 carbohydrate-containing feedstock many by-products are formed, comprising monomers, dimers, oligomers and polymers. These by-products are here and hereafter also called impurities. The by-products can form char and tar in the reaction solution. Char comprises insoluble compounds which can be removed via traditional solid liquid separation steps such as decanting, filtering and other solid liquid separation technologies. Tar is composed of low to high-molecular weight oligomeric and polymeric compounds and other high boiling compounds. Tar can be soluble and/or partly insoluble. Tar forms a problem during the isolation of levulinic acid, for example during a distillation step.
Other by-products are compounds with lower boiling points; for example formic acid and angelica lactone. The main part of these by-products preferably is separated from the levulinic acid to obtain composition 1.
After performing the acid catalyzed hydrolysis of the C6 carbohydrate-containing feedstock the reaction product X is purified. Reaction product X can be neutralized by a base before further treatment takes place. Filtration or centrifugation is typically used to separate by-products in reaction product X. Filtration can be performed after the reactor to remove the solid char and/or salts from the reaction product X. According to the prior art liquid-liquidextraction is often employed to separate at least a part of the by-products from the levulinic acid in reaction product X. Further, the reaction product X of levulinic acid can be purified, for example by distillation, solvent extraction and evaporation, for example flash evaporation, to obtain composition 1. The main part of the impurities is according to step b separated from reaction product X to obtain composition 1.

After purification of reaction product X in step b composition 1 is provided comprising at least 75 wt% levulinic acid, at most 15 wt% solvent and at most 10 wt% impurities, based on the total weight of composition1.
Preferably, the amount of levulinic acid in composition 1 is at least 85 wt%, more preferably at least 90 wt%, most preferably at least 95 wt%.
Preferably, the amount of solvent in composition 1 is at most 10 wt%, more preferably at most 7 wt%, most preferably at most 4 wt%.
Preferably, the amount of impurities in composition 1 is at most 5 wt%, more preferably at most 3 wt%, most preferably at most 1 wt%.
The solvent in the solution can be water or an organic solvent. Examples of suitable organic solvents are methyltetrahydrofuran (MTHF), methyl isoamyl ketone, methyl isobutyl ketone, diisobutyl ketone, acetophenone, cyclohexanone, isophorone, neopentyl alcohol, isoamyl alcohol, n-hexanol, n- heptanol, 2-ethyl hexanol, n-octanol, 1-nonanol, 1-undecanol, phenol, 4- methoxyphenol, methylene chloride, methyl isobutyl carbinol, anisol, ethylene glycol di-n-butyl ether, diethyl carbonate, methyl salicylate, methyl levulinate, ethyl levulinate, toluene, methyl-tertiary butyl ether, hexane, cyclohexane, chloro-benzene, dichloroethane, ortho- dichlorobenzene, 2,6-dimethyl cyclohexanone, tetrahydrofuran, furfural and mixtures thereof.

According to step c of the process according to the invention composition 1 is fed to a melt crystallization cycle. During melt crystallization levulinic acid is crystallized and melted to obtain composition 2. Composition 2 comprises a higher weight% of levulinic acid as composition 1 Composition 2 is isolated when the weight% of levulinic acid in composition 2 has a predetermined purity. A predetermined purity is a purity level of levulinic acid of 85-100 wt%, based on the total weight of composition 2. The person skilled in the art determines before the crystallization cycle is started which purity level the levulinic acid should be obtained. This purity level is here and hereafter defined as the predetermined purity.
When composition 2 does not have the predetermined purity the melt crystallization cycle can be repeated as many times as necessary to obtain as composition 2 levulinic acid with a predetermined purity.
A predetermined purity of composition 2 is, for example, a levulinic acid purity of at least 93 wt%, preferably a purity of at least 95 wt%, more preferably a purity of at least 98 wt%, in particular a purity of at least 99 wt%.

After obtaining composition 2 it is determined if composition 2 can be isolated or if it is necessary to repeat the melt crystallization cycle. For repeating the crystallization cycle composition 2 is fed to the melt crystallization cycle. The melt crystallization cycle comprises at least one melt crystallizer. When more than one melt crystallizer is present in the melt crystallization cycle the melt crystallizers can be placed in series or parallel. When the melt crystallization is repeated composition 2 can be fed to the same melt crystallizer as composition 1 or to one or more different melt crystallizers.
The melt crystallization cycle is repeated as many times as necessary to obtain as composition 2 levulinic acid with a predetermined purity.
Preferably, the melt crystallization cycle is repeated 0-6 times, more preferably 1-6 times, most preferably 1-4 times.
The process according to the invention can be a continuous, semi-continuous or a batch process.

Melt crystallization can be performed in different ways. Melt crystallization is well described in the literature for example in Jansens P.J. and Matsuoka M.: "Melt Crystallization", in: Encyclopedia of Separation Science, Eds. Wilson I.D., Adlard E.R., Cook M., and Poole C.F., Academic Press, San Diego, New York London, Sidney, Tokyo, (2000) 966-975 and in Arkenbout G.F., Melt crystallization technology", Technomic Publishing Company, Inc., Lancaster, Pennsylvania USA, (1995) 239-290.
An example of a melt crystallization process is layered growth melt crystallization. During layered growth melt crystallization the crystals grow as a layer on a cooled surface. The growth of the crystals is perpendicular to the surface. Industrial equipment for layered growth melt crystallization is normally operated batch-wise, whereby static and dynamic processes can be distinguished. In static processes the crystal growth occurs from a stagnant melt.
According to a preferred embodiment a full static melt crystallization cycle comprises 5 steps i-v as further described below.
In dynamic processes there is forced mixing of composition 1 during cooling in step i. Mixing is usually achieved by the circulation of composition 1 as a falling film or a flow over vertical heat-exchanger tubes, but other designs are also possible. The steps i-v as further described below are also needed to perform a dynamic melt crystallization process. Preferably, the melt crystallizer is a static crystallizer, more preferably at least one of the crystallizers that are used in the process according to the invention is a static crystallizer.

According to one embodiment of the invention the melt crystallization cycle comprises the following steps:
i. Feeding composition 1 to a melt crystallizer, wherein composition 1 is cooled to the freezing point or below the freezing point of levulinic acid to crystallize the levulinic acid in composition 1,
ii. Separating a liquid 1 from the crystallized levulinic acid,
iii. Purify the crystals obtained according to step i, after separation of liquid 1,
iv. Melting the crystals obtained according to step iii, after separation of liquid 1, to obtain a composition 2.
An example of such a process is suspension melt crystallization. During suspension melt crystallization the crystals form and grow while the crystals are suspended in composition 1. After crystal growth the crystals can be separated according to step iiby filtering or any other solid liquid separation technology.
The crystals can be purified according to step iii by either washing the crystals or subjecting the crystals to a sweating step as described below.

According to another embodiment of the invention the melt crystallization cycle comprises the following steps:
i. Feeding composition 1 to a melt crystallizer wherein composition 1 is cooled to at least one temperature Tc, wherein Tc (°C)=1.23.(W1) - (a value between 94.5 and 109.5) to crystallize the levulinic acid, wherein W1 is the weight% of levulinic acid in composition 1,
ii. Draining a liquid 1, wherein liquid 1 is 30-80 wt% of composition 1, based on the total weight of composition 1,
iii. Treating the crystals obtained in step i, after draining liquid 1, at at least one temperature Tw (°C)=1.23.(W1) - (a value between 84.5 and 101.5) to partly melt the crystals and to obtain a liquid 2, wherein W1 is the weight% of levulinic acid in composition 1,
iv. Draining liquid 2, wherein liquid 2 is 5-50 wt% of composition 1, based on the total weight of composition 1,
v. Melting the crystals obtained according to step iii, after draining of liquid 2, at at least one temperature Tm (°C)=1.23.(W1) - (a value lower than 84.5) to obtain a composition 2, wherein W1 is the weight% of levulinic acid in composition 1.

When composition 1 enters the melt crystallization cycle with a high purity and the crystallization takes place at a high temperature Tc composition 2 is obtained with a high purity. This means that the melt crystallization cycle does not have to be repeated many times to obtain the predetermined purity of composition 2.

According to step i composition 1 is fed to a melt crystallizer wherein the composition 1 is cooled to crystallize the levulinic acid. Composition 1 is cooled to the freezing point or below the freezing point of levulinic acid.

In the melt crystallizer composition 1 is cooled. Cooling can for example be performed by bringing composition 1 into contact with at least one cooling member. A cooling medium runs through the cooling member to cool the levulinic acid product below a certain temperature. A cooling member can be for example a cooling spiral, cooling plate or cooling jacket. Cooling can be performed by cocurrent and countercurrent circulation through the cooling member.

Cooling is performed to at least one temperature Tc. The temperature of the cooling can be constant or a temperature gradient which is gradually decreasing or increasing during a certain time period. The levulinic acid is cooled during a certain amount of time. The temperature determined during cooling is the temperature of composition 1. Cooling in step b can be performed for 5 minutes to 24 hours, preferably for 10 minutes to 20 hours, more preferably for 15 minutes to 10 hours.

After the crystals are formed the remaining liquid 1 is drained from the melt crystallizer or liquid 1 is separated from the crystallized levulinic acid., The amount of liquid 1 is 30-80 wt% of composition 1, based on the total weight of composition 1.

After draining of the liquid 1 the crystals in the crystallizer are treated at a temperature Tw (°C)=1.23.(W1) - (a value between 84.5 and 101.5) to partly melt the crystals and to obtain a liquid 2, wherein W1 is the weight% of levulinic acid in composition 1.
The treatment of the crystals at a raised temperature is referred to as sweating. During sweating part of the crystals including solvent and other impurities is melted and a liquid 2 is formed.

The temperature determined during sweating is the temperature of the cooling medium. Sweating can be performed at a fixed temperature or at a temperature gradient which is gradually decreasing or increasing during a certain time period.
Sweating can be performed for 5 minutes to 2 hours, preferably for 10 minutes to 1.5 hours, preferably for 15 minutes to 1 hour.
After treatment of the crystals the obtained liquid 2 is drained. The liquid 2 will comprise a certain amount of levulinic acid, but also solvent and other impurities. Liquid 2 can also be fed to another crystallizer to crystallize (part of) the remaining levulinic acid that is present in liquid 2. The amount of liquid 2 is 5-50 wt% of composition 1, based on the total weight of composition 1.
After separation of liquid 1 or after sweating and removal of the sweating liquid 2 the crystals are melted to obtain composition 2. Melting is performed by bringing the crystals to a temperature above the melting temperature of levulinic acid. Preferably, melting is performed at a temperature Tm (°C)=1.23.(W1) - (a value lower than 84.5) to provide a composition 2, wherein W1 is the weight% of levulinic acid in composition 1.
The temperature can be constant or a temperature gradient which is gradually decreasing or increasing during a certain time period. The temperature determined during melting is the temperature of the cooling medium.
Composition 2 is obtained. Composition 2 comprises a higher weight% of levulinic acid than composition 1.

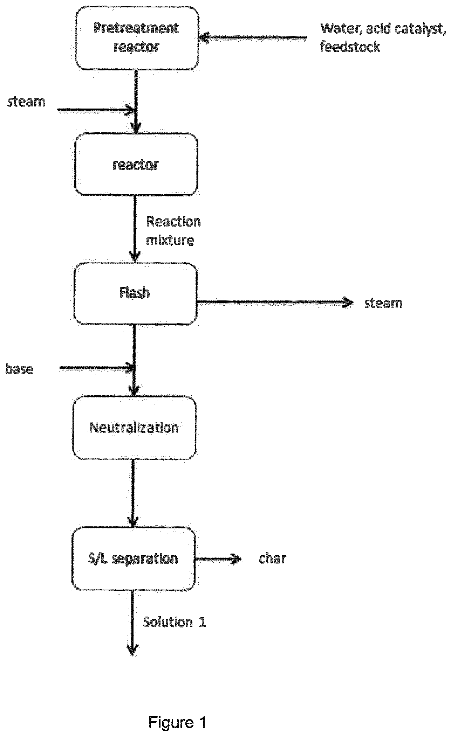

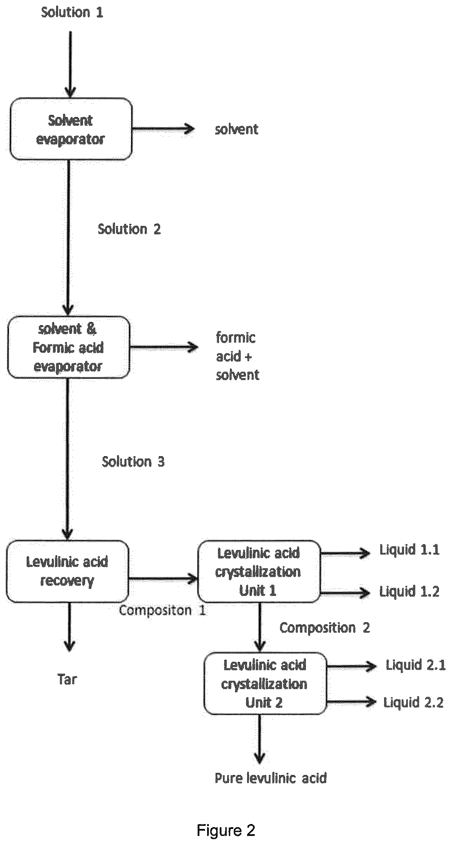

There is described herein (but not forming part of the present invention) an isolation section in a plant for the production of levulinic acid comprising at least one melt crystallizer.
The isolation section can be placed in the plant directly after evaporation or distillation. Composition 1 is obtained by purification of reaction product X. Composition 1 can, for example, be obtained during evaporation or distillation of reaction product X.
In the isolation section preferably the at least one crystallizer is a static melt crystallizer. In figure 1 and 2 a scheme of a plant according to one embodiment of the invention is shown.
In the plant according to figure 1 and 2 a continuous process for the production of levulinic acid can be performed.

According to figure 1 water, C6 carbohydrate-containing feedstock and an acid catalyst are mixed in a pretreatment reactor. The obtained mixture is fed to a reactor. The reaction mixture is brought to the desired temperature and pressure by steam. After the required reaction time the reaction mixture leaves the reactor and the pressure is reduced to atmospheric pressure in a flash tank. Steam is removed from the flash tank. Thereafter, the reaction mixture is neutralized by the addition of a base and a solid/liquid separation takes place to remove char. Solution 1 is obtained after solid/liquid separation.
In figure 2 the further purification of solution 1 is shown.
According to figure 2 solution 1 is fed to an isolation section formed by a series of three evaporation steps to obtain levulinic acid. During the first evaporation step solvent is removed and solution 2 is formed. Solution 2 is fed to the second evaporation step. During the second evaporation step formic acid and solvent are removed and solution 3 is formed. Solution 3 is fed to the third evaporation step. During the third evaporation step tar is removed and levulinic acid is obtained as the final product. This levulinic acid product is fed as composition 1 to a melt crystallization cycle comprising melt crystallizer 1 and melt crystallizer 2. In the melt crystallizer 1 the levulinic acid in composition 1 is crystallized and liquid 1.1 is drained. The crystals are objected to sweating and liquid 1.2 is obtained. After draining of liquid 1.2 the crystals are melted to obtain composition 2. Composition 2 is fed to melt crystallizer 2 for repeated melt crystallization. In the melt crystallizer 2 the levulinic acid in composition 2 is crystallized and liquid 2.1 is drained. The crystals are objected to sweating and liquid 2.2 is obtained. After draining of liquid 2.2 the crystals are melted to obtain a levulinic acid product with a predetermined purity.

The invention is further directed to the use of melt crystallization for the isolation of a levulinic acid composition, comprising at least 98 wt% levulinic acid, based on the total weight of the composition.

It is noted that the invention relates to all possible combinations of features described herein, preferred in particular are those combinations of features that are present in the claims.

It is further noted that the term 'comprising' does not exclude the presence of other elements. However, it is also to be understood that a description on a product comprising certain components also discloses a product consisting of these components. Similarly, it is also to be understood that a description on a process comprising certain steps also discloses a process consisting of these steps.
The invention will now be explained by way of the following examples without however being limited thereto.

### Example 1

1000 grams of 90.2 wt% of a levulinic acid (LA) solution that was obtained from the overhead of an evaporator, was fed to a laboratory scale static melt crystallizer. The static melt crystallizer was a jacketed glass vessel (volume 5 I.) equipped with a stainless steel U-tube for cooling. The U-tube was submerged in the feed solution.

The first crystallization step was carried out at 10 °C for 6 hours. The temperature was determined in the levulinic acid solution. After the crystal growth phase, the liquid was drained from the crystals. The crystals, attached to the wall of the U-tube, were subjected to a sweating phase in order to remove the impurities. The sweating phase lasted for 3 hours at a temperature of 20 °C and the sweating liquid 1.2 was drained. The temperature of 20 °C was the temperature of the cooling medium.
After the sweating phase, the crystals were melted by bringing the temperature of the cooling medium in the U-tube to 40 °C. The crystals melted from the U-tube surface. 358 grams of melted levulinic acid crystals were obtained with a purity of 96.1 wt% LA and 2,7 wt% of water.

The 358 grams melt from the first crystallization step was subjected to a second crystallization step, carried out at 15 °C, also for 6 hours. The crystallization temperature was determined in the levulinic acid product. After draining the liquid 2.1 the crystals were subjected to sweating at 30 °C for a period of 3 hours and the sweating liquid 2.2 was drained. The temperature during sweating was the temperature of the cooling medium. The crystals were melted to yield 104 grams of melted crystals could be retrieved with a purity of 99%, Moreover the melt was colorless. The combined drained liquids 2.1 and 2.2 were 254 g and had a purity of 94.9%.

The combined drained liquids 1.1 and 1.2 (642 grams) from the first crystallization step was subjected to a third crystallization step, performed at 10 °C for 16 hours. This liquid had an LA concentration of 86.8 wt%. The temperature during crystallization was the temperature of the liquid. After the third crystallization step the not crystallized liquid 3.1 was drained. The crystals were subjected to sweating phase of 2 hours at 15 °C, followed by 3 hours at 20 °C and the sweating liquid 3.2 was drained. The temperature during sweating was the temperature of the cooling medium. After melting the crystals a liquid of 284 g was obtained at a purity of 96% and 3% of water. The combined drained liquids 3.1 and 3.2 were 358g with a levulinic acid purity of 79.5%.

### Example 2

931 grams of a 97.5 wt% solution of levulinic acid was obtained via fractional distillation and consequently from the overhead of an evaporator. The solution was fed to a laboratory scale static melt crystallizer. The static melt crystallizer was a jacketed glass vessel (volume 5 I.) equipped with a stainless steel U-tube for cooling. The U-tube was submerged in the feed solution.

The crystallization step was carried out at 20 °C for 4 hours. The temperature was determined in the levulinic acid solution. After the crystal growth phase, the liquid 1.1 was drained from the crystals. The crystals, attached to the wall of the U-tube, were subjected to a sweating phase in order to remove the impurities. The sweating phase lasted for 3 hours at a temperature of 30 °C and the sweating liquid 1.2 was drained. The sweating temperature of 30 °C was the temperature of the cooling medium.
After the sweating phase, the crystals were melted by bringing the temperature of the cooling medium in the U-tube to 40 °C. The crystals melted from the U-tube surface. 230 grams of melted levulinic acid crystals were obtained with a purity of 99.8 wt% LA and 0.2 wt% of water. The combined drained liquids 1.1 and 1.2 (701 grams) from the crystallization step had a concentration of 96.7% of levulinic acid. By using one crystallization step a highly pure levulinic acid sample was obtained.

### Example 3

45.36 kg of a 79.8 wt% levulinic acid (LA) and 3.69 wt% formic acid (FA) solution was obtained from the overhead of an evaporator, and was fed in 8 batches to a laboratory scale static melt crystallizer (Volume 5 I). The static melt crystallizer was a jacketed glass vessel equipped with a stainless steel U-tube for cooling. The U-tube was submerged in the feed solution.

The first crystallization step was carried out at 2 °C for 8 hours. The temperature was determined in the levulinic acid solution. After the crystal growth phase, the liquid 1.1 was drained from the crystals. The crystals, attached to the wall of the U-tube, were subjected to a sweating phase in order to remove the impurities. The sweating phase lasted for 3 hours at a temperature of 15 °C and the sweating liquid 1.2 was drained. The temperature of 15 °C was the temperature of the cooling medium.
After the sweating phase, the crystals were melted by bringing the temperature of the cooling medium in the U-tube to 35 °C. The crystals melted from the U-tube surface. The collected melted crystals had a total weight of 11.3 kg with a purity of 90.3 wt% LA and 1.3 wt% of FA.
The combined sweat liquid 1.2 was 10.17 kg with 83 wt% LA and FA concentration of 2 wt%. The combined drained liquid 1.1 was 23.9 kg with a LA concentration of 73.5 wt% and FA concentration of 5.5 wt%.

The second crystallization step was carried out in 3 batches in the 5l static lab crystallizer at 10 °C for 6 hours. The temperature was determined in the levulinic acid solution. After the crystal growth phase, the liquid 2.1 was drained from the crystals. The crystals, attached to the wall of the U-tube, were subjected to a sweating phase in order to remove the impurities. The sweating phase lasted for 3 hours at a temperature of 20 °C and the sweating liquid 2.2 was drained. The temperature of 20 °C was the temperature of the cooling medium.
After the sweating phase, the crystals were melted by bringing the temperature of the cooling medium in the U-tube to 40 °C. The crystals melted from the U-tube surface. The collected melted crystals had a total weight of 3.96 kg with a purity of 93.8 wt% LA and 0.5 wt% of FA.

The combined sweat liquid 2.2 was 3.6 kg with 90.5 wt% LA and FA concentration of 1.1 wt%. The combined drained liquid 2.1 was 3.79 kg with a LA concentration of 86.5 wt% and FA concentration of 2.3 wt%.

The third crystallization step was carried out in 1 batch in the 5l static lab crystallizer at 15 °C for 6 hours. The temperature was determined in the levulinic acid solution. After the crystal growth phase, the liquid 3.1 was drained from the crystals. The crystals, attached to the wall of the U-tube, were subjected to a sweating phase in order to remove the impurities. The sweating phase lasted for 3 hours at a temperature of 25 °C and the sweating liquid 3.2 was drained. The temperature of 25 °C was the temperature of the cooling medium.
After the sweating phase, the crystals were melted by bringing the temperature of the cooling medium in the U-tube to 40 °C. The crystals melted from the U-tube surface. The melted crystals had a total weight of 1.12 kg with a purity of 99.3 wt% LA and 0.04 wt% of FA.
The sweat liquid 3.2 was 1.34 kg with 95.1 wt% LA and FA concentration of 0.2 wt%. The drained liquid 3.1 was 0.94 kg with a LA concentration of 85.4 wt% and FA concentration of 1.8 wt%.

After 3 crystallization steps a more than 99 wt% pure levulinic acid sample was obtained from a 79 wt% pure levulinic acid feedstock and the amount of formic acid in the sample was reduced from more than 3 wt% to less than 0.1 wt%.

## Claims

1. Process for the isolation of levulinic acid with a predetermined purity comprising the following steps:
a. Performing acid catalyzed hydrolysis of a C6 carbohydrate-containing feedstock to obtain reaction product X,
b. Purifying of reaction product X to provide a composition 1, comprising at least 75 wt% levulinic acid, at most 15 wt% solvent and at most 10 wt% impurities, based on the total weight of composition 1,
c. Feeding composition 1 to a melt crystallization cycle,
d. Repeating the melt crystallization cycle as many times as necessary to obtain as composition 2 levulinic acid with the predetermined purity, and
e. Isolating the levulinic acid with the predetermined purity,
Wherein a predetermined purity is a purity level of levulinic acid of 85-100 wt%, comprising at most 1 wt% of formic acid, at most 1 wt% of angelica lactone, at most 15 wt% of solvent and at most 5 wt% of impurities, based on the total weight of the levulinic acid.

2. Process according to claim 1, wherein the melt crystallization cycle comprises the following steps:
i. Feeding composition 1 to a melt crystallizer, wherein composition 1 is cooled to the freezing point or below the freezing point of levulinic acid to crystallize the levulinic acid in composition 1,
ii. Separating a liquid 1 from the crystallized levulinic acid,
iii. Purify the crystals obtained according to step i, after separation of liquid 1,
iv. Melting the crystals obtained according to step iii, after separation of liquid 1, to obtain a composition 2.

3. Process according to claim 1, wherein the melt crystallization cycle comprises the following steps:
i. Feeding composition 1 to a melt crystallizer wherein composition 1 is cooled to at least one temperature Tc, wherein Tc (°C)=1.23.(W1) - (a value between 94.5 and 109.5) to crystallize the levulinic acid, wherein W1 is the weight% of levulinic acid in composition 1,
ii. Draining a liquid 1, wherein liquid 1 is 30-80 wt% of composition 1, based on the total weight of composition 1,
iii. Treating the crystals obtained in step i, after draining liquid 1, at at least one temperature Tw (°C)=1.23.(W1) - (a value between 84.5 and 101.5) to partly melt the crystals and to obtain a liquid 2, wherein W1 is the weight% of levulinic acid in composition 1,
iv. Draining liquid 2, wherein liquid 2 is 5-50 wt% of composition 1, based on the total weight of composition 1,
v. Melting the crystals obtained according to step iii, after draining of liquid 2, at at least one temperature Tm (°C)=1.23.(W1) - (a value lower than 84.5) to obtain a composition 2, wherein W1 is the weight% of levulinic acid in composition 1.

4. Process according to any one of claims 1-3, wherein the predetermined purity of levulinic acid in composition 2 is at least 98 wt%, based on the total weight of composition 2.

5. Process according to any one of claims 2-4, wherein the melt crystallizer is a static melt crystallizer.

6. Process according to any one of claims 1-5, wherein the melt crystallization cycle is repeated 0-6 times.

7. Process according to any one of claims 1-5, wherein the melt crystallization cycle is repeated 1-4 times.

8. Process according to any one of claims 2-7, wherein the cooling in step i is performed for 5 minutes to 24 hours.

9. Process according to claim 3, wherein step iii is performed for 5 minutes to 2 hours.

10. Use of melt crystallization for the isolation of a levulinic acid composition, comprising at least 98 wt% levulinic acid, based on the total weight of the composition.

## Patentansprüche

1. Verfahren zur Isolierung von Levulinsäure mit einer vorbestimmten Reinheit, das die folgenden Schritte umfasst:
a. Durchführen einer säurekatalysierten Hydrolyse eines C6-Kohlenhydrat enthaltenden Ausgangsmaterials, um das Reaktionsprodukt X zu erhalten,
b. Reinigen des Reaktionsprodukts X zur Bereitstellung einer Zusammensetzung 1, die mindestens 75 Gew.-% Levulinsäure, höchstens 15 Gew.-% Lösemittel und höchstens 10 Gew.-% Verunreinigungen umfasst, bezogen auf das Gesamtgewicht der Zusammensetzung 1;
c. Einspeisen der Zusammensetzung 1 in einen Schmelzkristallisationszyklus,
d. Wiederholen des Schmelzkristallisationszyklus so oft wie nötig, um Levulinsäure mit der vorbestimmten Reinheit als Zusammensetzung 2 zu erhalten, und
e. Isolieren der Levulinsäure mit der vorbestimmten Reinheit,
wobei eine vorbestimmte Reinheit ein Reinheitsgrad von Levulinsäure von 85-100 Gew.-% ist, der höchstens 1 Gew.-% Ameisensäure, höchstens 1 Gew.-% Angelicalacton, höchstens 15 Gew.-% Lösemittel und höchstens 5 Gew.-% Verunreinigungen umfasst, bezogen auf das Gesamtgewicht der Levulinsäure.

2. Verfahren nach Anspruch 1, wobei der Schmelzkristallisationszyklus die folgenden Schritte umfasst:
i. Einspeisen der Zusammensetzung 1 in einen Schmelzkristallisator, wobei die Zusammensetzung 1 auf den Gefrierpunkt oder unter den Gefrierpunkt von Levulinsäure abgekühlt wird, um die Levulinsäure in der Zusammensetzung 1 zu kristallisieren.
ii. Trennen einer Flüssigkeit 1 von der kristallisierten Levulinsäure,
iii. Reinigen der gemäß Schritt i erhaltenen Kristalle nach Abtrennung von Flüssigkeit 1,
iv. Schmelzen der gemäß Schritt iii erhaltenen Kristalle nach Abtrennung von Flüssigkeit 1, um eine Zusammensetzung 2 zu erhalten.

3. Verfahren nach Anspruch 1, wobei der Schmelzkristallisationszyklus die folgenden Schritte umfasst:
i. Einspeisen der Zusammensetzung 1 in einen Schmelzkristallisator, wobei die Zusammensetzung 1 auf mindestens eine Temperatur Tc abgekühlt wird, wobei Tc (°C) = 1,23. (W1) - (ein Wert zwischen 94,5 und 109,5), um die Levulinsäure zu kristallisieren, wobei W1 die Gew.-% Levulinsäure in Zusammensetzung 1 darstellt,
ii. Ablassen einer Flüssigkeit 1, wobei Flüssigkeit 1 30-80 Gew.-% der Zusammensetzung 1 beträgt, bezogen auf das Gesamtgewicht der Zusammensetzung 1,
iii. Behandeln der in Schritt i erhaltenen Kristalle nach Ablassen der Flüssigkeit 1 bei mindestens einer Temperatur Tw (°C) = 1,23. (W1) - (ein Wert zwischen 84,5 und 101,5), um die Kristalle teilweise zu schmelzen und eine Flüssigkeit 2 zu erhalten, wobei W1 die Gew.-% Levulinsäure in Zusammensetzung 1 darstellt,
iv. Ablassen der Flüssigkeit 2, wobei Flüssigkeit 2 5-50 Gew.-% der Zusammensetzung 1 beträgt, bezogen auf das Gesamtgewicht der Zusammensetzung 1,
v. Schmelzen der gemäß Schritt iii erhaltenen Kristalle nach Ablassen der Flüssigkeit 2 bei mindestens einer Temperatur Tm (°C) = 1,23.(W1) - (ein Wert niedriger als 84,5), um eine Zusammensetzung 2 zu erhalten, wobei W1 die Gew.-% Levulinsäure in Zusammensetzung 1 darstellt.

4. Verfahren nach einem der Ansprüche 1-3, wobei die vorbestimmte Reinheit von Levulinsäure in Zusammensetzung 2 mindestens 98 Gew.-% beträgt, bezogen auf das Gesamtgewicht von Zusammensetzung 2.

5. Verfahren nach einem der Ansprüche 2-4, wobei der Schmelzkristallisator ein statischer Schmelzkristallisator ist.

6. Verfahren nach einem der Ansprüche 1-5, wobei der Schmelzkristallisationszyklus 0-6 Mal wiederholt wird.

7. Verfahren nach einem der Ansprüche 1-5, wobei der Schmelzkristallisationszyklus 1-4 Mal wiederholt wird.

8. Verfahren nach einem der Ansprüche 2-7, wobei das Abkühlen in Schritt i 5 Minuten bis 24 Stunden lang durchgeführt wird.

9. Verfahren nach Anspruch 3, wobei Schritt iii 5 Minuten bis 2 Stunden lang durchgeführt wird.

10. Verwendung der Schmelzkristallisation zur Isolierung einer Levulinsäurezusammensetzung, die mindestens 98 Gew.-% Levulinsäure umfasst, bezogen auf das Gesamtgewicht der Zusammensetzung.

## Revendications

1. Procédé d'isolement de l'acide lévulinique avec une pureté prédéterminée comprenant les étapes suivantes:
a. effectuer une hydrolyse à catalyse acide d'une charge alimentaire contenant un sucre en C6 pour obtenir le produit de réaction X
b. purifier le produit de réaction X pour donner une composition 1, comprenant au moins 75 % en poids d'acide lévulinique, au plus 15 % en poids de solvant et au plus de 10 % en poids d'impuretés, sur la base du poids total de la composition 1.
c. alimenter la composition 1 à un cycle de cristallisation à l'état fondu,
d. répéter le cycle de cristallisation à l'état fondu autant de fois que nécessaire pour obtenir comme composition 2 l'acide lévulinique avec la pureté prédéterminée, et
e. isoler l'acide lévulinique avec la pureté prédéterminée,
dans lequel une pureté prédéterminée est un niveau de pureté de l'acide lévulinique de 85 à 100 % en poids, comprenant au plus 1 % en poids d'acide formique, au plus 1 % en poids de lactone angélique, au plus 15 % en poids de solvant et au plus 5% en poids d'impuretés, sur la base du poids total de l'acide lévulinique.

2. Procédé selon la revendication 1, le cycle de cristallisation à l'état fondu comprenant les étapes suivantes:
i. alimenter la composition 1 à un cristallisoir pour état fondu, dans laquelle la composition 1 est refroidie au point de congélation ou au-dessous du point de congélation de l'acide lévulinique pour cristalliser l'acide lévulinique dans la composition 1,
ii. séparer un liquide 1 de l'acide lévulinique cristallisé,
iii. purifier les cristaux obtenus selon l'étape i, après séparation du liquide 1,
iv. fondre les cristaux obtenus selon l'étape iii, après séparation du liquide 1, pour obtenir la composition 2.

3. Procédé selon la revendication 1, le cycle de cristallisation à l'état fondu comprenant les étapes suivantes:
i. alimenter la composition 1 à un cristallisoir pour état fondu, dans laquelle la composition 1 est refroidie à au moins une température Tc , Tc (°C) = 1,23.(W1) - (une valeur comprise entre 94,5 et 109,5) pour cristalliser l'acide lévulinique, dans laquelle W1 est le % en poids de l'acide lévulinique dans la composition 1,
ii. drainer un liquide 1, dans laquelle le liquide 1 est de 30 à 80 % en poids de la composition 1, sur la base du poids total de la composition 1,
iii. traiter les cristaux obtenus dans l'étape i, après le drainage du liquide 1, à au moins une température Tw (°C) = 1,23.(W1) - (une valeur entre 84,5 et 101,5) pour fondre partiellement les cristaux et obtenir un liquide 2, dans laquelle W1 est le % en poids de l'acide lévulinique dans la composition 1,
iv. drainer le liquide 2, dans laquellele liquide 2 est de 5 à 50 % en poids de la composition 1, sur la base du poids total de la composition 1,
v. fondre les cristaux obtenus selon l'étape iii, après le drainage du liquide 2, à au moins une température Tm (°C) = 1,23.(W1) - (une valeur inférieure à 84,5) pour obtenir une composition 2, dans laquelle W1 est le % en poids de l'acide lévulinique dans la composition 1.

4. Procédé selon l'une quelconque des revendications 1 à 3, dans lequel la pureté prédéterminée de l'acide lévulinique dans la composition 2 est d'au moins 98 % en poids, sur la base du poids total de la composition 2.

5. Procédé selon l'une quelconque des revendications 2 à 4, dans lequel le cristallisoir pour état fondu est un cristallisoir pour état fondu statique.

6. Procédé selon l'une quelconque des revendications 1 à 5, dans lequel le cycle de cristallisation à l'état fondu est répété de 0 à 6 fois.

7. Procédé selon l'une quelconque des revendications 1 à 5, dans lequel le cycle de cristallisation à l'état fondu est répété de 1 à 4 fois.

8. Procédé selon l'une quelconque des revendications 2 à 7, dans lequel le refroidissement dans l'étape i est effectué durant 5 minutes à 24 heures.

9. Procédé selon la revendication 3, dans lequel l'étape iii est effectuée durant 5 minutes à 2 heures.

10. Utilisation de la cristallisation à l'état fondu pour l'isolement d'une composition d'acide lévulinique, comprenant au moins 98 % en poids d'acide lévulinique, sur la base du poids total de la composition.
